# NOUVEAU FASCICULE DE BREVET EUROPEEN

(11) **EP 1 250 328 B2**
(45) Date de publication et mention de la décision concernant l'opposition: **21.04.2010**
(45) Mention de la délivrance du brevet: 05.07.2006
(21) Numéro de dépôt: 01907700.7
(22) Date de dépôt: 25.01.2001
(51) Int. Cl.: C07D 251/10, C07D 251/52, C07D 251/72, A61P 3/10, A61K 31/53

(54) **DERIVES AMINES DE DIHYDRO-1,3,5-TRIAZINE ET LEURS APPLICATIONS EN THERAPEUTIQUE**
DIHYDRO-1,3,5-TRIAZIN-DIAMIN-DERIVATE UND IHRE THERAPEUTISCHE VERWENDUNG
DIHYDRO-1,3,5-TRIAZINE AMINE DERIVATIVES AND THEIR THERAPEUTIC USES

(30) Priorité: 26.01.2000 FR 0000996
(43) Date de publication de la demande: 23.10.2002
(73) Titulaire: Merck Sante, 69008 Lyon (FR)
(72) Inventeur: MOINET, Gérard, F-91400 Orsay (FR); CRAVO, Daniel, F-78500 Sartrouville (FR); DOARE, Liliane, F-91170 Viry Chatillon (FR); KERGOAT, Micheline, F-91440 Bures-sur-Yvette (FR); MESANGEAU, Didier, F-77380 Combs la Ville (FR)
(74) Mandataire: Bernasconi, Jean Raymond
(86) Numéro de dépôt international: PCT/FR2001/000241
(87) Numéro de publication internationale: WO 2001/055122

(56) Documents cités:
- WO-A-99/31088
- JP-A- 48 064 088
- JP-B2- 61 041 915
- US-A- 3 287 366
- DATABASE WPI Section Ch, Week 197911 Derwent Publications Ltd., London, GB; Class B03, AN 1979-20599B XP002150918 -& JP 54 014986 A (TAIHO PHARM CO LTD), 3 février 1979 (1979-02-03) cité dans la demande
- DATABASE CHEMABS [en ligne] CHEMICAL ABSTRACTS SERVICE, COLUMBUS, OHIO, US; HIRANO, KANEO ET AL: "6-Amino-1,4-dihydro-2-substituted amino-1,3,5-triazines" retrieved from STN Database accession no. 79:137200 XP002151148 & JP 48 064088 A (YAMANOUCHI PHARMACEUTICAL CO., LTD.) 5 septembre 1973 (1973-09-05)
- "JOURNAL OF MEDICINAL CHEMISTRY,US,AMERICAN CHEMICAL SOCIETY. WASHINGTON" JOURNAL OF MEDICINAL CHEMISTRY,US,AMERICAN CHEMICAL SOCIETY. WASHINGTON, vol. 6, no. 4, 1963, pages 370-378, XP002128704 ISSN: 0022-2623
- STEVENS, M. F. G. ET AL: "Introduction of a triflate group into sterically hindered positions in 1-aryl-4,6-diamino-1,3,5-triazines and their Dimroth rearrangement products" JOURNAL OF HETEROCYCLIC CHEMISTRY., vol. 30, no. 4, 1993, pages 849-853, XP002151147 HETEROCORPORATION. PROVO., US ISSN: 0022-152X
- MODEST, EDWARD J.: 'Chemical and Biological Studies on 1,2-Dihydro-s-triazines.' THE JOURNAL OF ORGANIC CHEMISTRY vol. 21, no. 1, 19 Janvier 1956, pages 1 - 13
- MODEST, EDWARD J.: 'Chemical and Biological Studies on 1,2-Dihydro-s-triazines' THE JOURNAL OF ORGANIS CHEMISTRY vol. 21, no. 1, 19 Janvier 1956, pages 14 - 20
- REMBARZ, GERHARD ET AL: 'Reaktionen mit Natriumdicyanimid' JOURNAL FÜR PRAKTISCHE CHEMIE vol. 4, no. 26, pages 314 - 318
- WALSH, ROGER J. A. ET AL: 'The Structure Activity Relationship of Antibacterial Substituted 1-Phenyl-4,6-Diamino-1,2-Dihydro-2,2-Dimeth yl-s-triazines' EUROPEAN JOURNAL OF MEDICAL CHEMISTRY vol. 12, no. 6, pages 495 - 500

## Description

La présente invention conceme des dérivés aminés de dihydro-1,3,5-triazine utiles dans le traitement de pathologies associées au syndrome d'insulinorésistance.

Des dérivés aminés de dihydro-1,3,5-triazine ayant des propriétés hypogtycémiantes ont été décrits dans JP-A-73 64 088 et JP-A-79 14 986.

Des composés triazines ont également été décrits dans JP54014986, JP48064088, J. Med. Chem. 6,4,1963, 370-378, J. Het Chem. 30,4,1993, 849-853, US 3,287,366, J. Org. Chem. vol 21, n° 1, 1956, 1-13; J. Org. Chem. vol 21, 1956, 14-20; Journal für Praktische Chemie, 4. Reihe, 26 (5-6), 1964, 314-318; Eur, J. Med. Chem. 12(6), 1977, 495-500.
J. Org. Chem. 21, 1956, 1-13 décrit le composé (IIe) mais ne concerne cependant pas le diabète. JP 48 064 088 ne décrit pas spécifiquement de composés selon la formule générale (I) de la présente revendication 1.

La présente invention vise à fournir de nouveaux composés ayant des propriétés améliorées.

La présente invention a ainsi pour objet un composé de formule générale (I) : 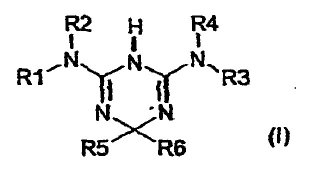 dans laquelle:
R1 et R2 sont choisis indépendamment parmi les groupes :
   - alkyle (C1-C20) éventuellement substitué par halogène, alkyle (C1-C5), alkoxy (C1-C5), cycloalkyle (C3-C8),
   - alkylène (C2-C20) éventuellement substitué par halogène, alkyle (C1-C5), alkoxy (C1-C5)
   - alkyne (C2-C20) éventuellement substitué par halogène, alkyle (C1-C5), alkoxy (C1-C5)
   - cycloalkyle (C3-C8) éventuellement substitué par alkyle (C1-C5), alkoxy (C1-C5)
   - hétérocycloalkyle (C3-C8) portant un ou plusieurs hétéroatomes choisis parmi N, O, S et substitué ou non par alkyle (C1-C5), alkoxy (C1-C5)
   - aryl (C6-C14) alkyle (C1-C20) éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   - aryle (C6-C14) éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   - hétéroaryle (C1-C13) portant un ou plusieurs hétéroatomes choisis parmi N, O, S et éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
R3 et R4 représentent un atome d'hydrogène ;
R5 et R6 sont choisis indépendamment parmi les groupes :
   -H,
   -alkyle (C1-C20) éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   alkylène (C2-C20) éventuellement substitué par amino, hydroxy, thio, halogène, alkyle (C1-C5), alkoxy (C1-C5), atkylthio (C1-C5), alkylamino (C1-C5), aryl (C6-C14) oxy; aryl (C6-C14) alkoxy (C1-C5), cyano, trifluorométhyle, carboxy, carboxyméthyle ou carboxyéthyle,
   alkyne (C2-C20) éventuellement substitué par amino, hydroxy, thio, halogène, alkyle (C1-C5), alkoxy (C1-C5), altrylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle.
   -cycloalkyle (C3-C8) éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   -hétérocycloalkyle (C3-C8) portant un ou plusieurs hétéroatomes choisis parmi N, O, S et éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   -aryle (C6-C14) éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
   - aryl (C6-C14) alkyle(C1-C5) éventuellement substitué par amino, hydroxy, thio, halogène, alkyle (C1-C5). atkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,

R5 et R6 pouvant former avec l'atome de carbone sur lequel ils sont fixés un cycle à m chainons (m compris entre 3 et 8) comprenant ou non un ou plusieurs hétéroatomes choisis parmi N, O, S et éventuellement substitué par amino, hydroxy, thio, halogène, alkyle (C1-C5), atkoxy (C1-C5), alkylthio (C1-C5), alkylamino (C1-C5), aryl (C6-C14) oxy, aryl (C6-C14) alkoxv (C1-C5), cyano, trifluorométhyle, carboxy, carboxyméthyle ou carboxyéthyle,
ou pouvant former avec l'atome de carbone un reste polycyclique en C10-C30 éventuellement substitué par atom, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
l'atome d'azote d'un groupe hétérocycloalkyle ou hétéroaryle pouvant être substitué par un groupe alkyle (C1-C5), cycloalkyle (C3-C8), aryle(C6-C14), aryl(C6-C14)alkyle(C1-C5) ou acyle(C1-C6), ainsi que les formes tautomères, énantiomères, diastéréoisomères et épimères et les sels pharmaceutiquement acceptables, à l'exception du composé pour lequel R1 = phényle, R2 = méthyle, R3 = R4 = H et R5 = R6 = méthyle.

Par cycle à m chaînons formé par R5 et R6, on entend en particulier un cycle saturé tel qu'un groupe cyclohexyle, pipéridinyle ou tétrahydropyrannyie.

Par groupe polycyclique formé par R5 et R6, on entend un groupe polycyclique carboné éventuellement substitué et en particulier un reste de stéroïde.

Un groupe particulier de composés de formule (I) est celui dans laquelle R5 est l'hydrogène.

Un autre groupe particulier de composés de formule (I) est celui dans laquelle R5 et R6 forment avec l'atome de carbone sur lequel ils sont fixés un cycle à m chainons, (m compris entre 3 et 8) comprenant ou non un ou plusieurs hétéroatomes choisis parmi N, O, S et pouvant être substitué par un ou plusieurs groupements suivants : alkyle (C1-C5), amino, hydroxy, alkylamino(C1-C5), alkoxy(C1-C5), alkylthio(C1-C5); aryle(C6-C14), aryl(C6-C14)-alkoxy(C1-C5).
ou forment avec l'atome de carbone un reste polycyclique en C10-C30 substitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle.

Un autre groupe particulier de composés de formule (I) est celui dans laquelle R5 et R6 sont choisis indépendamment parmi les groupes :
- alkyle (C1-C20) sustitué ou non par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyte ou carboxyéthyle.

Plus particulièrement, un groupe préféré de composés de formule (I) est celui dans laquelle R1 et R2 sont un groupe alkyle, avantageusement méthyle.

L'invention se rapporte également aux formes tautomères, aux énantiomères, diastéréoisomères et épimères des composés de formule générale (I).

Les composés de formule générale (I) possèdent des atomes d'azote basiques qui peuvent être monosalifiés ou disalifiés par des acides organiques ou minéraux.

Les composés de formule générale (I) peuvent être préparés par réaction d'un composé de formule générale (II) 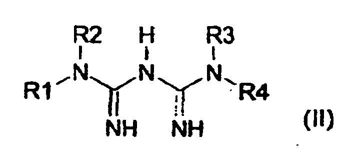 dans laquelle R1, R2, R3 et R4 sont définis tels que précédemment, avec un composé de formule général (III), (IV) ou (V) 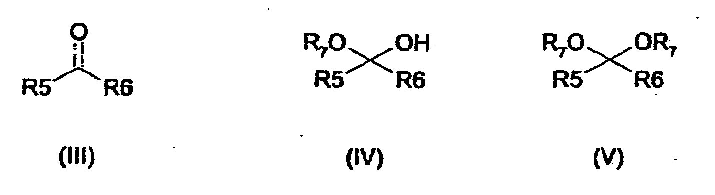 dans lesquelles R5 et R6 sont définis tels que précédemment et R7 est un groupement méthyle ou éthyle, dans un solvant polaire (par exemple éthanol ou diméthyformamide) en présence d'un acide organique (par exemple l'acide camphorsulfonique) ou minéral (par exemple l'acide chlorhydrique).

Les composés de formule générale (II) sont des biguanides dont la synthèse est maîtrisée par tous les hommes de l'art. Nous citons pour exemple certaines publications décrivant la synthèse de tels composés (FR 1537604, FR 2132396 ; K. H. Slotta and R. Tschesche, Ber., 1929(62b), 1398 ; S. L. Shapiro, V. A. Parrino, E. Rogow and L. Freedman, J. Org. Chem., 1959(81), 3725 ; S. L. Shapiro, V. A. Parrino, and L. Freedman, J. Org. Chem., 1959(81), 3728 ; S. L. Shapiro, V. A. Parrino, and L. Freedman, J. Org. Chem., 1959(81), 4636).

Les composés selon la présente invention sont utiles dans le traitement des pathologies associées au syndrome d'insulinorésistance (syndrome X). L'insulinorésistance se caractérise par une réduction de l'action de l'insuline (cf. Presse Médicale, 1997, 26(n°14), 671-677) et est impliquée dans un nombre important d'états pathologiques, tel que le diabète et plus particulièrement le diabète non-insulino-dépendant (diabète de type II ou NIDDM), la dyslépidémie, l'obésité, l'hypertension artérielle, ainsi que certaines complications microvasculaires et macrovasculaires comme l'athérosclérose, les rétinopathies et les neuropathies.

A ce sujet, on se rapportera par exemple à Diabètes, vol 37, 1988, 1595-1607 ; Journal of Diabetes and its complications, 1998, 12, 110-119 ou Horm. Res., 1992, 38, 28-32.

Notamment les composés de l'invention présentent une forte activité hypoglycémiante.

Les composés selon la présente invention sont également utilisables pour traiter les complications chroniques qui sont notamment dues à la formation "d'advanced glycosylation end-products" noté AGE'S, issus de la réaction de glycoxydation entre le glucose, ses dérivés d'oxydation et les fonctions amino des protéines, dont les réactions dites de Maillard de glycation du glyoxal par exemple.

En effet, des données récentes de la littérature montrent clairement l'impact des AGE's sur les complications rénales (Nephr. Dial. Transplant., 2000, 15 (suppl 2). 7-11), sur l'athérosclérose, la maladie d'Alzheimer et autres maladies de neurodégénérescence (Glycoconj. J., 1998, 15(10), 1039-42 ; Brain Res., 2001, 888(2), 256). La formation d'AGE peut également jouer un rôle important dans la pathogénèse de l'angiopathie, notamment chez les diabétiques, ainsi que dans la sénilité (J. Neuropathol. Exp. Neurol., 2000, 59(12), 1094).

La présente invention a donc également pour objet des compositions pharmaceutiques comprenant, à titre de principe actif, un composé selon l'invention.

Ces compositions pharmaceutiques sont particulièrement destinées à traiter le diabète, des pathologies dues à la formation d'AGE's, telles que notamment les complications rénales, l'athérosclérose, l'angiopathie, la maladie d'Alzheimer, les maladies neurodégénératives et la sénilité.

Les composés pharmaceutiques selon l'invention peuvent être présentés sous des formes destinées à l'administration par voie parentérale, orale, rectale, permuqueuse ou percutanée.

Elles seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les dérivés de la cellulose ou de la cellulose microcristalline, les carbonates alcalino-terreux, le phosphate de magnésium, les amidons, les amidons modifiés, le lactose pour les formes solides.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes (0.5 mg à 1000 mg) en fonction de l'indication thérapeutique et de la voie d'administration, ainsi que de l'âge et du poids du sujet.

A titre d'exemple, voici quelques biguanides de formule II utilisés dans la synthèse de dérivés de formule I.

**TABLEAU I**

| | **formule** | **sel** | **Pf en °C (Köfler)** |
|---|---|---|---|
| A | 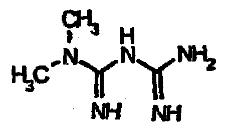 | HCl | 223-225 |
| B | 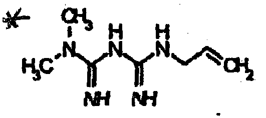 | HCl | 176-178 |
| C | 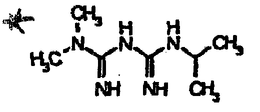 | HCl | 230-232 |
| D | 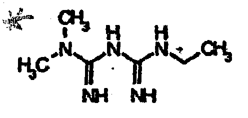 | HCl | 210-212 |
| E | 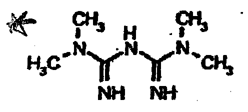 | HCl | 254-256 |
| F | 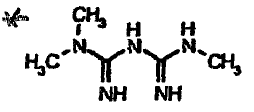 | HCl | 158-160 |
| G | 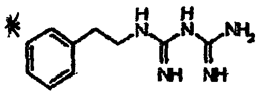 | HCl | 100-102 |

| | | | |
|---|---|---|---|
| * Composés ne faisant pas partie de l'invention telle que revendiquée. | | | |

Les exemples suivants illustrent la préparation des composés de formule I.

### EXEMPLE 1

### Synthèse du chlorhydrate de 2-amino-3,6-dihydro-4-diméthylamino-6-éthyl-1,3,5-triazine*

A une solution de composé A (25.7 g ; 0.155 mol dans 200 mL de DMF sont additionnés 23 mL de propionaldéhyde et 3.6 g d'acide camphorsulfonique. Après 2 heures de reflux, le solvant est éliminé sous vide et 100 mL d'acétonitrile sont ajoutés. Le solide formé est essoré et séché (21.9 g ; 69%).
F = 218-220°C
RMN ¹H (DMSO-d6, 200 MHz) : 1.10 (t, 3H) ; 1.80 (m, 2H) ; 3.20 (s, 6H) ;4.83 (m, 1H) ; 7.57 (m, 2H) ; 8.65 (s, 1H) ; 8.90 (s, 1H)
RMN ¹³C (DMSO-d6, 50 MHz) : 6.41 (CH3) ; 27.59 (CH2) ; 35.64 (CH3) ; 60.75 (CH) ; 155.01 (C=N) ; 156.67 (C=N)

### EXEMPLE 2

### Synthèse du chlorhydrate de 2,4-bis-diméthylamino-3,6-dihydro-6-méthyl-1,3,5-triazine

A une solution de composé E (41.10 g, 0.212 mol) dans 200 mL d'éthanol absolu sont ajoutés 61 mL d'acétal et 5 g d'acide camphorsulfonique. Le tout est porté au reflux pendant 72 heures puis concentré. Le brut est trituré avec de l'acétonitrile et le solide formé est essoré puis recristallisé dans de l'acétonitrile. 24 g (51.5%) d'un solide sont obtenus.
F = 200-202°C
* Composés ne faisant pas partie de l'invention telle que revendiquée.
RMN ¹H (DMSO-d6, 200 MHz) : 1.34 (d, 3H) ; 3.02 (s, 6H) ; 4.72 (m, 1 H) ;4.83 (m, 1 H) ; 8.80 (s, 2H)
RMN ¹³C (DMSO-d6, 50 MHz) : 22.59 (CH3) ; 37.76 (CH3) ; 59.02 (CH) ; 156.35 (C=N)

Les caractéristiques de ces composés et d'autres composés de formule I sont données dans le tableau II ci-après :

**TABLEAU II**

| | **formule** | **sel** | **Pf en °C (Köfler)** | **RMN ¹³C 50.32 MHz** |
|---|---|---|---|---|
| 1 | 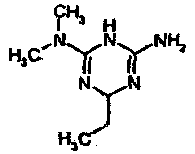 | HCl | 218-220 | DMSO-d6 6.41, CH3 27.59, CH2 35.64, 2 CH3 60.75, CH 155.01, 156.67, 2 Cquaternaire |
| 2 | 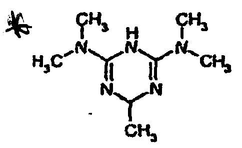 | HCl | 200-202 | DMSO-d6 22.58, 37.75, 5 CH3 59.01, CH 156.34, 2 Cquaternaire |
| 3 | 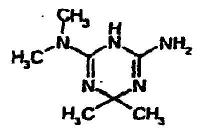 | | 193-195 | DMSO-d6 32.06, 37.40, 2 CH3 67.85, 158.16, 3 Cquaternaire |
| 4 | 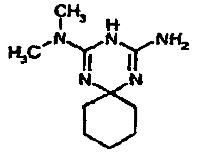 | HCl | 243-245 | DMSO-d6 21.66, 25.19, 37.72, 3 CH2 37.89, 2 CH3 67.51, 156.83, 158.24, 3 Cquaternaire |
| 5 | 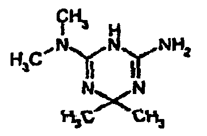 | méthanesulfo nate | 174-176 | DMSO-d6 34.31, 41.36, 44.79, 5 CH3 69.75, 160.30, 161.44, 3 Cquaternaire |
| 6 | 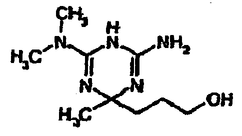 | | 138-140 | DMSO-d6 28.04, CH2 30.84, 37:40, 3 CH3 42.06, 62.24, 2 CH2 70.00, 158.24, 158.69, 3 Cquaternaire |
| 7 | 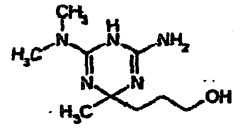 | HCl | 150-152 | DMSO-d6 27.39, CH2 28.78, 39.14, 3 CH3 40.21, 61.30, 2 CH2 68.46, 156.48, 157.84, 3 Cquaternaire |
| 8 | 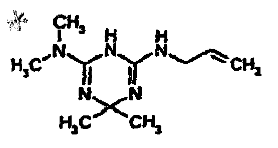 | HCl | 124-126 | DMSO 28.95, 38.65,2 CH3 42.77, CH2 69.75, Cquaternaire 115.93, CH2 149.12, CH 155.70. 156.16. 2 Cquaternaire |
| 9 | 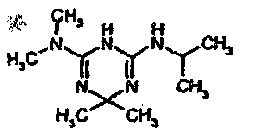 | HCl | 149-151 | DMSO-d6 26.20, 32.39, 40.73, 6 CH3 46.16, CH 60.09, 158.83, 159.14, 3 Cquaternaire |
| 10 | 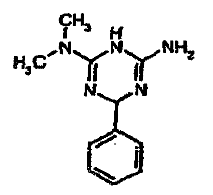 | HCl | 239-241 | DMSO-d6 37.78, 2 CH3 62.39, CH 126.66, 129.47, 5 CH 141.87, 156.52, 158.38, 3 Cquaternaire |
| 11 | 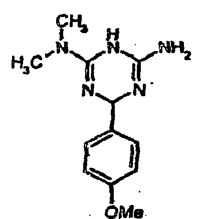 | HCl | 221-223 | DMSO-d6 37.23, 55.60, 3 CH3 61.88, CH 114.32, 127.66, 4 CH 133.17, 156.11, 157.86, 159.93, 4 Cquaternaire |
| 12 | 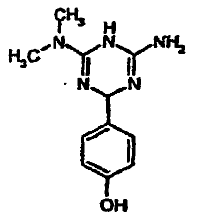 | HCl | 251-253 | DMSO-d6 37.75, 2 CH3 62.67, 116.16, 128.16, 5 CH 131.72, 156.64, 158.31, 158.88, 4 Cquaternaire |
| 13 | 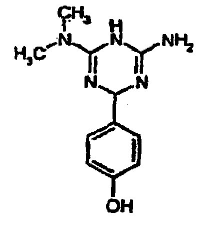 | | >260 | DMSO-d6 39.55, 39.71, 2 CH3 65.92, 117.71, 130.17, 5 CH 131.72, 156.64, 158.31, 158.88, 4 Cquaternaire |
| 14 | 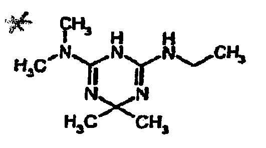 | furnarate | 172-174 | 15.48, 29.33, 3 CH3 35.68, CH2 37.43, 2 CH3 65.71, Cquaternaire 135.47,2 CH 156.21, 156.63, 168.35, 4 Cquaternaire |
| 15 | 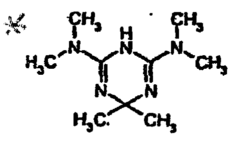 | HCl | 250-252 | DMSO-d6 28.74, 37.38, 6 CH3 66.53, 155.28, 3 Cquaternaire |
| 16 | 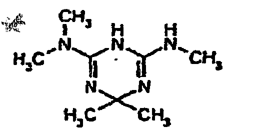 | HCI | 183-185 | DMSO-d6 32.62, 40.96, 5 CH3 69.37, 159.30, 160.19, 3 Cquaternaire |
| 17 | 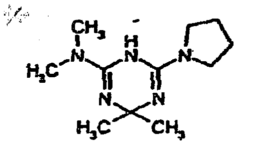 | HCl | >260 | DMSO-d6 22.78, 2 CH3 28.96, 2 CH2 40.13,2 CH3 42.73, 2 CH2 65.63, 155.42, 155.71, 3 Cquaternaire |
| 18 | 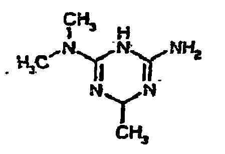 | HCI | 229-231 | DMSO-d6 22.97, 37.76, 3 CH3 58.59, CH 57.85, 159.39, 2 Cquaternaire |
| 19 | 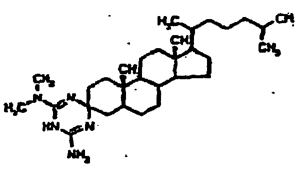 | HCl | >260 | Spectre succinct DMSO-d6 69.06, 159.78, 161.17, 3 Cquaternaire |
| 21 | 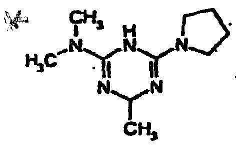 | carbonate | >140 | DMSO-d6 20.51. CH3 24.73, 25.39, 2 CH2 39.98, 2 CH3 46.44, 47.91, 2 CH2 58.49, CH 154.58, 156.63, 160.61, 3 Cquaternaire |
| 22 | 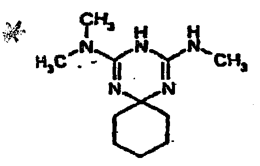 | HCl | >260 | DMSO-d6 21.18, 24.68, 3 CH2 27.26, CH3 37.00, 2 CH2 37.37, 2 CH3 67.12, 155.89, 156.86. 3 Cquaternaire |
| 23 | 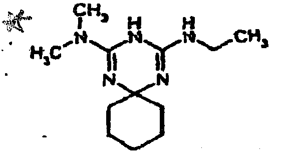 | HCl | 248-250 | DMSO-d6 5.11, CH321.17, 24.70, 35.39, 37.04, 6 CH2 37.36, 2 CH3 67.09, 155.90, 156.21, 3 Cquaternaire |
| 24 | 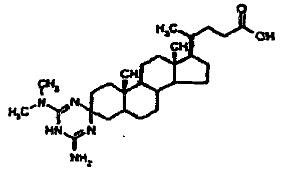 | HCl | >260 | Spectre succinct DMSO-d6 67.46, 68.80, 156.76, 157.47, 157.99, 159.14. 3 Cquaternaire 175.90, 176.11, COOH |
| 25 | 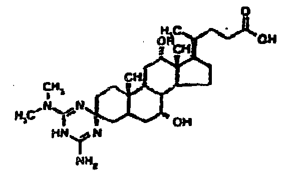 | HCl | >260 | Spectre succinct DMSO-d6 64.87, 69.85, 2 CHOH 66.55, 154.91, 156.19, 3 Cquaternaire 173.75, COOH |
| 26 | 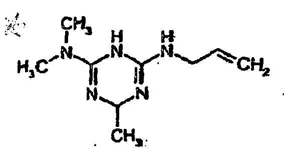 | HCl | 91-93 | DMSO-d6 25.76, 37.28, 3 CH3 43.28, CH2 64.27, CH 115.21, CH2 137.55, CH 159.79, 160.77. 2 Cquaternaire |
| 27 | 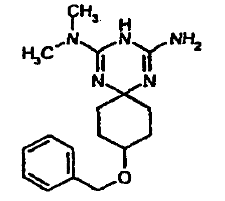 | HCl | >260 | DMSO-d6 25.69, 27.25, 4 CH2 39.13, 2 CH3 67.25, Cquaternaire 70.01, CH2 72.50, CH 128.17, 128.34, 129.07, 5 CH 139.79, 156.81, 158.30, 3 Cquaternaire |
| 28 | 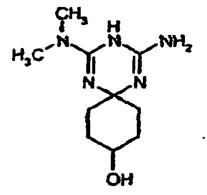 | HCl | >250 | DMSO-d6 29.83, 34.4, 4 CH2 38.83, 2 CH3 66.17, CH 67.06, 156.25, 157.28, 3 Cquaternaire |
| 29 | 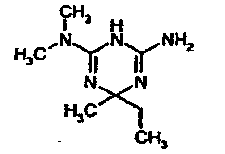 | carbonate | 133-135 | DMSO-d6 7.25, 26.81, 2 CH3 34.32, CH2 37.17, 2 CH3 68.59, 156.46, 157.71, 160.78, 4 C quaternaire |
| 30 | 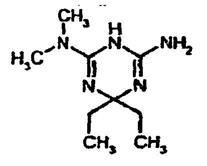 | carbonate | 140-144 | 8.68, 2CH3 34.54, 2 CH2 37.91, 2 CH3 74.98, 157.84, 159.14, 160.82, 4 Cquaternaire |
| 31 | 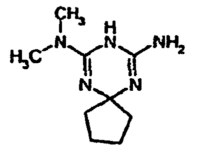 | HCl | 207-209 | DMSO-d6 22.50, 2 CH2 38.00, 2 CH3 39.78. 2 CH2 75.51, 157.18, 158.37, 3 Cquaternaire |
| 32 | 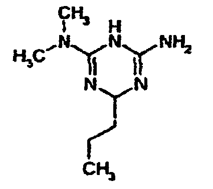 | carbonate | Se décompose | DMSO-d6 14.55, CH3 17.20, CH2 37.45, 2 CH3 39.00, CH2 62.43, CH 157.52, 159.04, 160.65, 3 Cquaternaire |
| 33 | 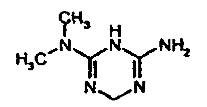 | HCl | >260 | D20 37.90. 2 CH3 48.69, CH2 154.82, 156.33, 2 Cquaternaire |
| 34 | 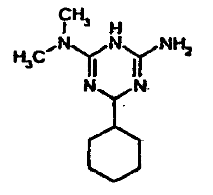 | paratoluènesu lfonate | 201-203 | DMSO-d6 21.65, CH3 25.95, 26.07, 26.58, 26.89, 27.50, 5 CH2 37.56, 2 CH3 44.74, 66.56, 126.32, 129.08, 6 CH 138.99, 145.86, 158.18, 156.86, 4 Cquaternaire |
| 35 | 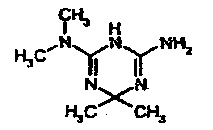 | HCl | 157-159 | DMSO-d6 29.10, 37.86, 4 CH3 65.90, 154.82, 156.33, 3 Cquaternaire |
| 36 | 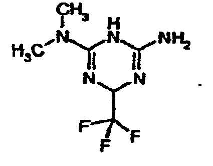 | paratoluènesu lfonate | 251-253 | DMSO-d6 21.14, 37.26, 3 CH3 114.80, 120.70, 126.41, 132.12, CF3 125.82. 128.54, 4 CH 138.37, 145.49, 155.78, 157.18, 4 Cquaternaire |
| 37 | 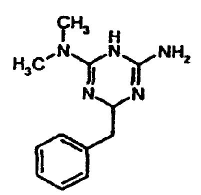 | paratoluènesu lfonate | 159-161 | DMSO-d6 21.17, 36.95, 3 CH3 42.60, CH2 62.10, 126.86, 127.21, 128.55, 128.63, 130.32, 10 CH 135.14, 138.30, 145.67, 156.18, 157.44, 5 Cquaternaire |
| 38 | 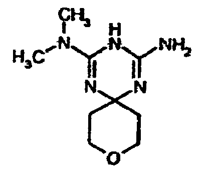 | HCl | >260 | DMSO-d6 37.41, 2 CH3 37.47, 62.73, 4 CH2 64.76, 156.35, 157.77, 3 Cquaternaire |
| 39 | 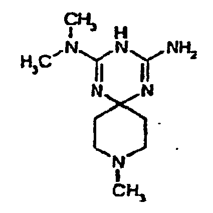 | HCl | >260 | DMSO-d6 34.12, 2 CH2 38.63, 42.60, 3 CH3 48.72, 2 CH2 64.01, 156.11, 157.78, 3 Cquaternaire |
| 41 | 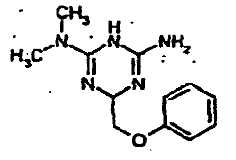 | paratoluènesulfonate | 194-196 | DMSO-d6 21.17, 37.03, 3 CH3 6037, CH 70.05, CH2 115.08, 121.60, 125.84, 128-54, 12995, 10 CH 138.28, 145.64, 156.40, 157.70, 158.45, 5 Cquaternaire |
| 42 | 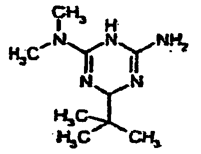 | HCl | >260 | DMSO-d6 24.12, 37.15, 5 CH3 39.90, Cquaternaire 68.39, CH 156.57, 158.10, 2 Cquaternaire |
| 43 | 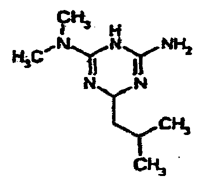 | HCl | Se décompose | DMSO-d6 22.95, 23.05, 2 CH3 25.87, CH 36.94, 2 CH3 45.71, CH2 62.38, CH 157.15, 157.42, 158.34, 3 Cquaternaire |
| 44 | 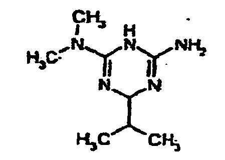 | HCl | 213-215 | DMSO-d6 15.99, 17.12, 2 CH3 3457, CH 37.17, 2 CH3 65.68, CH 156.45, 158.12, 2 Cquaternaire |
| 45 | 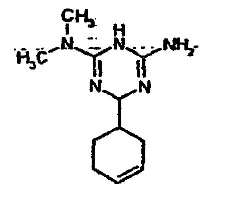 | paratoluenesulfonate | 217-219 | DMSO-d6 21.17, CH3 22.53, 24.48, 25.30, 3 CH2 37.20, 2 CH3 40.07, 64.37, 2 CH 125.68, 125.83, 127.19, 128.61, 6 CH 138.53, 145.24, 156.06, 157.36, 4 Cquaternaire |

| | | | | |
|---|---|---|---|---|
| * Composés ne faisant pas partie de l'invention telle que revendiquée. | | | | |

On donnera ci-après des résultats des études pharmacologiques.

### ETUDE DE L'ACTIVITE ANTIDIABETIQUE CHEZ LE RAT NOSTZ

On a déterminé l'activité antidiabétique des composés de formule (I) par voie orale sur un modèle expérimental de diabète non insulinodépendant, induit chez le rat par la Streptozotocine.

Le modèle de diabète, non insulinodépendant, est obtenu chez le rat par une injection néonatale (le jour de la naissance) de streptozotocine.

Les rats diabétiques utilisés sont âgés de 8 semaines. La stabulation des animaux est réalisée, du jour de leur naissance au jour de l'expérimentation, dans une animalerie à température régulée de 21 à 22°C et soumise à un cycle fixe de lumière (de 7 à 19 h) et d'obscurité (de 19 à 7 h). Leur alimentation a consisté en un régime d'entretien, eau et nourriture ont été fournies «ab libitum», à l'exception du jeûne de 2 heures précédent les tests où la nourriture est retirée (état postabsorptif).

Les rats sont traités par voie orale pendant un (J1) ou quatre (J4) jours avec le produit à tester. Deux heures après la demière administration du produit et 30 minutes après anesthésie des animaux au Pentobarbital sodique (Nembutal® ), un prélèvement sanguin de 300 µL est effectué à l'extrémité de la queue.

A titre d'exemple sont rassemblés dans le tableau III des résultats obtenus. Ces résultats montrent l'efficacité des composés de formule (I) pour faire diminuer la glycémie chez les animaux diabétiques. Ces résultats sont exprimés en pourcentage d'évolution de la glycémie à J1 et J4 (nombre de jours de traitement) par rapport à J0 (avant le traitement).

**TABLEAU III**

| composés | 20 mg/kg/J | | 200 mg/kg/J | |
|---|---|---|---|---|
| | J1 | J4 | J1 | J4 |
| 1 | -7 | -2 | -13 | -15 |
| *2 | -11 | -10 | -12 | -12 |
| 3 | -10 | -8 | -18 | -22 |
| 4 | 0 | -1 | -20 | -10 |
| 7 | -8 | -11 | -10 | -16 |
| *15 | -8 | -9 | -4 | -5 |
| *17 | -12 | -8 | -8 | -14 |
| 18 | -6 | -4 | -29 | -28 |
| 19 | -10 | -6 | -4 | -14 |
| *21 | -7 | -2 | -21 | -24 |
| *22 | -23 | -16 | -13 | 0 |
| 25 | -4 | -11 | -7 | -6 |
| *26 | -6 | -11 | -14 | -9 |
| 27 | -14 | -9 | -12 | -13 |
| 28 | -4 | -1 | -4 | -13 |
| 31 | -5 | -11 | -3 | -15 |
| 32 | 2 | 0 | -22 | -18 |
| 33 | -7 | -6 | -9 | -14 |
| 34 | -5 | -15 | -6 | -21 |
| 37 | -7 | -8 | -10 | -15 |
| 39 | -6 | -6 | -4 | -7 |
| 40 | -8 | -12 | -18 | -18 |
| 42 | -5 | -4 | -26 | -17 |
| 43 | -4 | -16 | -12 | -17 |
| 44 | -7 | -6 | -22 | -25 |

| | | | | |
|---|---|---|---|---|
| * Composés ne faisant pas partie de l'invention telle que revendiquée. | | | | |

### ETUDE DE L'ACTIVITE ANTIGLYCATION

Les composés (I) sont aussi capables d'inhiber les réactions dites de Maillard par «effet de captation» sur les dérivés α-dicarbonilés tel que le glyoxal - c'est l'effet antiglycation. Cet effet inhibiteur de la réaction de Maillard par les composés selon l'invention a été étudié *in vitro* par dosage des cétamines ("fructosamines") produites lors de l'incubation de l'albumine avec le méthylglyoxal en présence ou non d'un composé de formule (I) selon l'invention.

Une solution d'albumine bovine à 6,6 mg/ml dans du tampon phosphate 0,2 M pH 7,4 est incubée avec du méthylglyoxal 1 mM en présence ou non d'un composé selon l'invention à une concentration de 10 mM. L'incubation est faite dans des conditions stériles à 37°C pendant 6 jours. A la fin de la période d'incubation, la quantité de cétamines est mesurée avec un kit de dosage de fructosamine commercialement disponible (kit «FRA», référence produit : 0757055, produits Roche S.A) suivant les instructions du fabricant.

A titre d'exemple sont rassemblés dans le tableau IV des résultats obtenus dans ces conditions expérimentales : taux de fructosamine après incubation de l'albumine avec le méthylglyoxal en présence des composés (I) selon l'invention par rapport au taux de fructosamine quand l'albumine est incubée avec le méthylglyoxal en absence des composés (I) selon l'invention.

**TABLEAU IV**

| Composés (I) | Baisse du taux de fructosamines (%) |
|---|---|
| 1 | 62 |
| 10 | 80 |
| 11 | 89 |
| 12 | 90 |
| 13 | 95 |
| 18 | 69 |
| 33 | 79 |
| 34 | 64 |
| 36 | 66 |
| 37 | 65 |
| 40 | 66 |
| 43 | 68 |
| 45 | 67 |

## Revendications

1. Composés de formule générale (I) 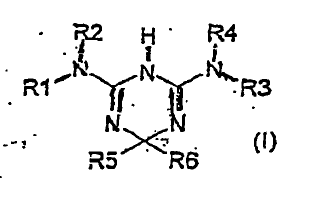 dans laquelle:
R1 et R2 sont choisis indépendamment parmi les groupes :
- alkyle (C1-C20) éventuellement substitué par halogène, alkyle (C1-C5), alkoxy (C1-C5), cycloalkyle (C3-C8),
- alkylène (C2-C20) éventuellement substitué par halogène, alkyle (C1-C5), alkoxy (C1-C5)
- alkyne (C2-C20) éventuellement substitué par halogène, alkyle (C1-C5), alkoxy (C1-C5)
- cycloalkyle (C3-C8) éventuellement substitué par alkyle (C1-C5), alkoxy (C1-C5)
- hétérocycloalkyle (C3-C8) portant un ou plusieurs hétéroatomes choisis parmi N, O, S éventuellement substitué par alkyle (C1-C5), alkoxy (C1-C5)
- aryl (C6-C14) alkyle (C1-C20) éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- aryle (C6-C14) éventuellement substitué par amino, hydroxy, thio, halogéne, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- hétéroaryle (C1-C13) portant un ou plusieurs hétématomes choisis parmi N, O, S et éventuellement substitué par amino, hydroxy, thio, hafogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
R3 et R4 représentent un atome d'hydrogène ;
R5 et R6 sont choisis indépendamment parmi les groupes :
- H,
- alkyle (C1-C20) éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- alkylène (C2-C20) éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- alkyne (C2-C20) éventuellement substitué par amino, hydroxy, thio, halogène, alkyle (C1-C5), alkoxy (C1-C5), alkylthio (C1-C5), alkylamino (C1-C5), aryl (C5-C14) oxy, aryl (C6-C14) alkoxy (C1-C5), cyano, trifluorométhyle, carboxy, carboxyméthyle ou carboxyéthyle,
- cycloalkyle (C3-C8) éventuellement substitué par amino, hydroxy, thio, halogène, alkyle (C1-C5), alkoxy (C1-C5), alkylthio (C1-C5), alkylamino (C1-C5), aryl (C5-C14) oxy, aryl (C6-C14) alkoxy (C1-C5), cyano, trifluorométhyle, carboxy, carboxyméthyle ou carboxyéthyle,
- hétérocycloalkyle (C3-C8) portant un ou plusieurs hétéroatomes choisis parmi N, O, S et éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméihyle ou carboxyéthyle,
- aryle (C6-C14) éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
- aryl (C6-C14) alkyle(C1-C5) éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
R5 et R6 pouvant former avec l'atome de carbone sur lequel ils sont fixés un cycle à m chainons (m compris entre 3 et 8) comprenant ou non un ou plusieurs hétérostomes choisis parmi N, O, S et éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyethyte,
ou pouvant former avec l'atome de carbone un reste polycyclique en C10-C30 éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle,
l'atome d'azote d'un groupe hétérocycloalkyle ou hétéroaryle pouvant être substitué par un groupé alkyle (C1-C5), cycloalkyle (C3-C8), aryle(C6-C14), aryl(C6-C14)alkyle(C1-C5) ou aryle(C1-C6), ainsi que les formes tautomères, énantiomères, diastéréoisomères et épimères et les sels pharmaceutiquement acceptables, à l'exception du composé par lequel R1 = phényle, R2 = méthyle, R3 = R4 = H et R5 = R6 = méthyle..

2. Composés de formule (I) selon la revendication 1, dans laquelle R5 est l'hydrogène.

3. Composés de formule (I) selon la revendication 1, dans laquelle R5 et R6 forment avec l'atome de carbone sur lequel ils sont fixés un cycle à m chainons. (m compris entre 3 et 8) comprenant ou non un ou plusieurs hétéroatomes choisis parmi N, O, S et éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy- (C1-C5), cyano, trifluorométhyle, carboxy, carboxyméthyle ou carboxyéthyle,
ou forment avec l'atoms de carbone un reste polycyclique en C10-C30 éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyl ou carboxyéthyle.

4. Composés de formule (I) selon la revendication 1, dans laquelle R5 est un groupe alkyle (C2-C20) substitué par amino, hydroxy, thio, halogène, alkyle (C1-C5), alkoxy (C1-C5), alkylthio (C1-C5), alkylamino (C1-C6), aryl (C6-C14) oxy, aryl (C6-C14) alkoxy (C1-C5), cyano, trifluorométhyle, carboxy, carboxyméthyle ou carboxyéthyle.

5. Composé de formule (I) selon la revendication 1 dans lequelle R5 et R6 sont choisis parmi H et/ou des groupes alkyle (C1-C20) éventuellement substitué par amino, hydroxy, thio, halogène, alkyle (C1-C5), alkoxy (C1-C5), alkylthio (C1-C5), alkylamino (C1-C5), aryl (C6-C14) oxy, aryl (C6-C14) alkoxy (C1-C5), cyano, trifluorométhyle, carboxy, carboxyméthyle ou carboxyéthyle.

6. Composés de formule (I) selon l'une quelconque des revendications 1 à 5, dans laquelle R1 et R2 sont un groupe méthyle et R3 et R4 représentent un hydrogène.

7. Composés de formule (I) selon l'une quelconque des revendications 1 à 6, dans laquelle R1 et R2 sont un groupe méthyle, R3 et R4 représentent un atome d'hydrogène, R5 représente un atome d'hydrogène et R6 représente un groupe méthyle,
ainsi que les formes tautomères, énantiomères, diastéréoisomères et épimères, et les sels pharmaceutiquement acceptable.

8. Procédé de préparation d'un composé selon la revendication 1 comprenant la réaction d'un composé de formule générale (II) 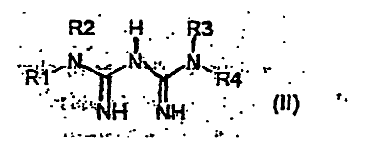 dans laquelle R1. R2. R3 et R4 sont définis tels que précédemment, avec un composé de formule générale (III), (IV) ou (V) 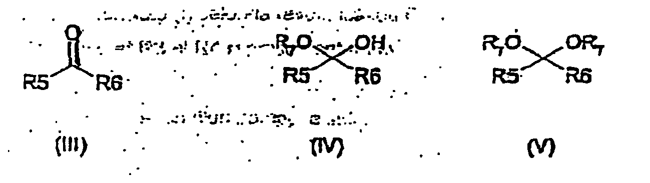 dans lesquelles R5 et R6 sont définis tels que précédemment et R7 est un groupement méthyle ou éthyle, dans un solvant polaire en présence d'un acide organique ou minéral.

9. Composition pharmaceutique comprenant, à titre de principe actif, un composé selon l'une quelconque des revendications 1 à 7.

10. Utilisation d'un composé selon l'une quelconque des revendications 1 à 7, pour la fabrication d'un médicament destiné au traitement des pathologies associées au syndrome d'insulinorésistance.

11. Utilisation d'un composé selon l'une quelconque des revendications 1 à 7, pour la fabrication d'un médicament destiné au traitement du diabète.

12. Utilisation d'un composé selon l'une quelconque des revendications 1 à 7. pour la fabrication d'un médicament destiné au traitement des pathologies dues à la formation d'AGE's.

13. Utilisation d'un composé selon l'une quelconque des revendications 1 à 7. pour la fabrication d'un médicament destiné au traitement de pathologies cholsies parmi les complications rénales, l'athérosclérose, l'angiopathie, la maladie d'Alzheimer, les maladies neurodégénératives et la sénilité.

## Claims

1. Compounds of the general formula (I) 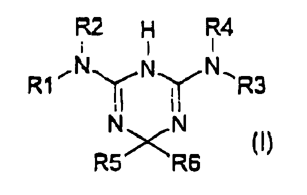 in which:
R1 and R2 are independently selected from among the groups:
- (C1-C20) alkyl, optionally substituted by halogen, (C1-C5) alkyl, (C1-C5) alkoxy, (C3-C8) cycloalkyl,
- (C2-C20) alkylene, optionally substituted by halogen, (C1-C5) alkyl, (C1-C5) alkoxy,
- (C2-C20) alkyne, optionally substituted by halogen, (C1-C5) alkyl, (C1-C5) alkoxy,
- (C3-C8) cycloalkyl, optionally substituted by (C1-C5) alkyl, (C1-C5) alkoxy,
- (C3-C8) heterocycloalkyl bearing one or more heteroatoms selected from among N, O, S and optionally substituted by (C1-C5) alkyl, (C1-C5) alkoxy,
- (C6-C14) aryl (C1-C20) alkyl, optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
- (C6-C14) aryl, optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
- (C1-C13) heteroaryl bearing one or more heteroatoms selected from among N, O, S and optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
R3 and R4 represent a hydrogen atom;
R5 and R6 are independently selected from among the groups:
- H,
- (C1-C20) alkyl, optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
- (C2-C20) alkylene, optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
- (C2-C20) alkyne, optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
- (C3-C8) cycloalkyl, optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
- (C3-C8) heterocycloalkyl bearing one or more heteroatoms selected from among N, O, S and optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
- (C6-C14) aryl, optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
- (C6-C14) aryl (C1-C5) alkyl, optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
R5 and R6 being capable of forming with the carbon atom to which they are attached a cycle with m chain links (m between 3 and 6) which may or may not comprise one or more heteroatoms selected from among N, O, S and optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
or being capable of forming with the carbon atom a C10-C30 polycyclic residue optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
the nitrogen atom of a heterocycloalkyl or heteroaryl group possibly being substituted by a (C1-C5) alkyl, (C3-C8) cycloalkyl, (C6-C14) aryl, (C6-C14) aryl (C1-C5) alkyl, or (C1-C6) aryl group, together with the tautomeric, enantiomeric, diastereoisomeric and epimeric forms thereof and pharmaceutically acceptable salts, with the exception of the compound in which R1 = phenyl, R2 = methyl, R3 = R4 = H and R5 = R6 = methyl.

2. Compounds of the formula (I) according to claim 1, in which R5 is hydrogen.

3. Compounds of the formula (I) according to claim 1, in which R5 and R6 form with the carbon atom to which they are attached a cycle with m chain links (m between 3 and 8) which may or may not comprise one or more heteroatoms selected from among N, O, S and optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl,
or form with the carbon atom a C10-C30 polycyclic residue, optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl.

4. Compounds of the formula (I) according to claim 1, in which R5 is a (C2-C20) alkyl group substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl.

5. A compound of the formula (I) according to claim 1 in which R5 and R6 are selected from among H and/or (C1-C20) alkyl groups optionally substituted by amino, hydroxy, thio, halogen, (C1-C5) alkyl, (C1-C5) alkoxy, (C1-C5) alkylthio, (C1-C5) alkylamino, (C6-C14) aryloxy, (C6-C14) aryl (C1-C5) alkoxy, cyano, trifluoromethyl, carboxy, carboxymethyl or carboxyethyl.

6. Compounds of the formula (I) according to any one of claims 1 to 5, in which R1 and R2 are a methyl group and R3 and R4 represent a hydrogen.

7. Compounds of the formula (I) according to any one of claims 1 to 6, in which R1 and R2 are a methyl group, R3 and R4 represent a hydrogen atom, R5 represents a hydrogen atom and R6 represents a methyl group, together with the tautomeric, enantiomeric, diastereoisomeric and epimeric forms thereof, and pharmaceutically acceptable salts.

8. A method of preparing a compound according to claim 1 comprising the reaction of a compound of the general formula (II) 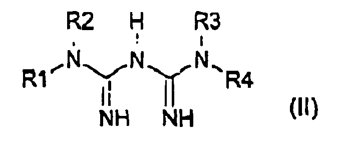 in which R1, R2, R3 and R4 are defined as previously, with a compound of the general formula (III), (IV) or (V) 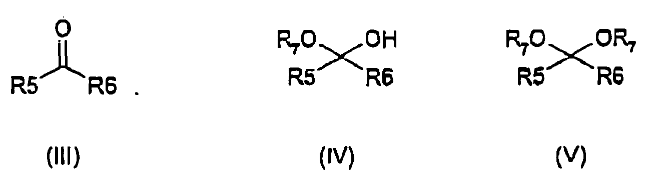 in which R5 and R6 are defined as previously and R7 is a methyl or ethyl group, in a polar solvent in the presence of an organic or inorganic acid.

9. A pharmaceutical composition comprising, as active ingredient, a compound according to any one of claims 1 to 7.

10. Use of a compound according to any one of claims 1 to 7, for the manufacture of a medicine intended for the treatment of pathological conditions associated with insulin resistance syndrome.

11. Use of a compound according to any one of claims 1 to 7, for the manufacture of a medicine intended for the treatment of diabetes.

12. Use of a compound according to any one of claims 1 to 7, for the manufacture of a medicine intended for the treatment of pathological conditions due to the formation of AGEs.

13. Use of a compound according to any one of claims 1 to 7, for the manufacture of a medicine intended for the treatment of pathological conditions selected from among kidney complications, atherosclerosis, angiopathy, Alzheimer's disease, neurodegenerative diseases and senility.

## Patentansprüche

1. Verbindungen mit der allgemeinen Formel (I) 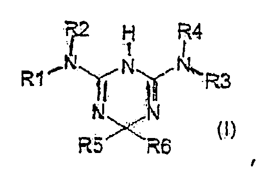 in welcher:
- R₁ und R₂ unabhängig voneinander ausgewählt sind aus den Gruppen:
- (C₁- bis C₂₀-)Alkyl, das gegebenenfalls mit Halogen, (C₁-bis C₅-)Alkyl, (C₁- bis C₅-)Alkoxy und (C₃- bis C₈-)Cycloalkyl substituiert ist,
- (C₂- bis C₂₀-)Alkenyl, das gegebenenfalls mit Halogen, (C₁- bis C₅-)Alkyl und (C₁- bis C₅-)Alkoxy substituiert ist,
- (C₂- bis C₂₀-)Alkinyl, das gegebenenfalls mit Halogen, (C₁- bis C₅-)Alkyl und (C₁- bis C₅-)Alkoxy substituiert ist,
- (C₃- bis C₈-)Cycloalkyl, das gegebenenfalls mit (C₁- bis C₅-)Alkyl und (C₁- bis C₅-)Alkoxy substituiert ist,
- (C₃- bis C₈-)Heterocycloalkyl, das ein oder mehrere Heteroatome trägt, die aus N, O und S ausgewählt sind, und gegebenenfalls mit (C₁- bis C₅-)Alkyl und (C₁- bis C₅-)Alkoxy substituiert ist,
- (C₆- bis C₁₄-)Aryl(C₁- bis C₂₀-)alkyl, das gegebenenfalls mit Amino, Hydroxy, Thio, Halogen, (C₁- bis C₅-)Alkyl, (C₁- bis C₅-)Alkoxy, (C₁- bis C₅)Alkylthio, (C₁- bis C₅-)Alkylamino, (C₆- bis C₁₄-)Aryloxy, (C₆- bis C₁₄-)Aryl(C₁- bis C₅-)alkoxy, Cyano, Trifluormethyl, Carboxy, Carboxymethyl oder Carboxyethyl substituiert ist,
- (C₆- bis C₁₄-)Aryl, das gegebenenfalls mit Amino, Hydroxy, Thio, Halogen, (C₁- bis C₅-) Alkyl, (C₁- bis C₅-) Alkoxy, (C₁- bis C₅-)Alkylthio, (C₁- bis C₅-)Alkylamino, (C₆- bis C₁₄-)Aryloxy, (C₆- bis C₁₄-) Aryl(C₁- bis C₅-)alkoxy, Cyano, Trifluormethyl, Carboxy, Carboxymethyl oder Carboxyethyl substituiert ist, und
- (C₁- bis C₁₃-)Heteroaryl, das ein oder mehrere Heteroatome trägt, die aus N, O und S ausgewählt sind, und gegebenenfalls mit Amino, Hydroxy, Thio, Halogen, (C₁- bis C₅-) Alkyl, (C₁- bis C₅-)Alkoxy, (C₁- bis C₅-)Alkylthio, (C₁- bis C₅-)Alkylamino, (C₆- bis C₁₄-)Aryloxy, (C₆- bis C₁₉-) Aryl (C₁- bis C₅-)Alkoxy, Cyano, Trifluormethyl, Carboxy, Carboxymethyl oder Carboxyethyl substituiert ist, und
- R₃ und R₄ ein Wasserstoffatom bedeuten.
- R₅ und R₆ unabhängig voneinander ausgewählt sind aus den Gruppen:
- H,
- (C₁- bis C₂₀-)Alkyl, das gegebenenfalls mit Amino, Hydroxy, Thio, Halogen, (C₁- bis C₅-)Alkyl, (C₁- bis C₅-)Alkoxy, (C₁- bis C₅-)Alkylthio, (C₁- bis C₅-)Alkylamino, (C₆- bis C₁₄-)Aryloxy, (C₆- bis C₁₄-)Aryl(C₁- bis C₅-)alkoxy, Cyano, Trifluormethyl, Carboxy, Carboxymethyl oder Carboxyethyl substituiert ist,
- (C₂- bis C₂₀-)Alkenyl, das gegebenenfalls mit Amino, Hydroxy, Thio, Halogen, (C₁- bis C₅-)Alkyl, (C₁- bis C₅-)Alkoxy, (C₁- bis C₅-)Alkylthio, (C₁- bis C₅-)Alkylamino, (C₆- bis C₁₄-)Aryloxy, (C₆- bis C₁₄-)Aryl(C₁- bis C₅-)alkoxy, Cyano, Trifluormethyl, Carboxy, Carboxymethyl oder Carboxyethyl substituiert ist,
- (C₂- bis C₂₀-)Alkinyl, das gegebenenfalls mit Amino, Hydroxy, Thio, Halogen, (C₁- bis C₅-)Alkyl, (C₁- bis C₅-)Alkoxy, (C₁- bis C₅-)Alkylthio, (C₁- bis C₅-)Alkylamino, (C₆- bis C₁₄-)Aryloxy, (C₆- bis C₁₄-)Aryl(C₁- bis C₅-)alkoxy, Cyano, Trifluormethyl, Carboxy, Carboxymethyl oder Carboxyethyl substituiert ist,
- (C₃- bis C₈-)Cycloalkyl, das gegebenenfalls mit Amino, Hydroxy, Thio, Halogen, (C₁- bis C₅-)Alkyl, (C₁- bis C₅-)Alkoxy, (C₁- bis C₅-)Alkylthio, (C₁- bis C₅-)Alkylamino, (C₆- bis C₁₄-)Aryloxy, (C₆- bis C₁₄-)Aryl(C₁- bis C₅-)alkoxy, Cyano, Trifluormethyl, Carboxy, Carboxymethyl oder Carboxyethyl substituiert ist,
- (C₃- bis C₈-) Heterocycloalkyl, das ein oder mehrere Heteroatome trägt, die aus N, O und S ausgewählt sind, und gegebenenfalls mit Amino, Hydroxy, Thio, Halogen, (C₁-bis C₅-)Alkyl, (C₁- bis C₅-)Alkoxy, (C₁- bis C₅-)Alkylthio, (C₁- bis C₅-)Alkylamino, (C₆- bis C₁₄-)Aryloxy, (C₆- bis C₁₉-)*Aryl (C₁- bis C₅-)alkoxy, Cyano, Trifluormethyl, Carboxy, Carboxymethyl oder Carboxyethyl substituiert ist,
- (C₆- bis C₁₄-)Aryl, das gegebenenfalls mit Amino, Hydroxy, Thio, Halogen, (C₁- bis C₅-)Alkyl, (C₁- bis C₅-)Alkoxy, (C₁- bis C₅-)Alkylthio, (C₁- bis C₅-)Alkylamino, (C₆- bis C₁₉-)Aryloxy, (C₆- bis C₁₄-) Aryl (C₁- bis C₅-)alkoxy, Cyano, Trifluormethyl, Carboxy, Carboxymethyl oder Carboxyethyl substituiert ist, und
- (C₆- bis C₁₄-)Aryl(C₁- bis C₅-)alkyl, das gegebenenfalls mit Amino, Hydroxy, Thio, Halogen, (C₁- bis C₅-)Alkyl, (C₁- bis C₅-)Alkoxy, (C₁- bis C₅-)Alkylthio, (C₁- bis C₅-)Alkylamino, (C₆- bis C₁₄-)Aryloxy, (C₆- bis C₁₄-)Aryl(C₁- bis C₅-)alkoxy, Cyano, Trifluormethyl, Carboxy, Carboxymethyl oder Carboxyethyl substituiert ist, und
- R₅ und R₆ mit dem Kohlenstoffatom, an welches sie gebunden sind, einen Cyclus mit m Gliedern (m gleich 3 bis 8) bilden können, der gegebenenfalls ein oder mehrere Heteroatome enthält, die aus N, O und S ausgewählt sind, und gegebenenfalls mit Amino, Hydroxy, Thio, Halogen, (C₁-bis C₅-)Alkyl, (C₁- bis C₅-)Alkoxy, (C₁- bis C₅-)Alkylthio, (C₁- bis C₅-)Alkylamino, (C₆- bis C₁₄-)Aryloxy, (C₆- bis C₁₄-)Aryl(C₁- bis C₅-) alkoxy, Cyano, Trifluormethyl, Carboxy, Carboxymethyl oder Carboxyethyl substituiert ist, oder mit dem Kohlenstoffatom einen polycyclischen C₁₀- bis C₃₀-Rest bilden können, der gegebenenfalls mit Amino, Hydroxy, Thio, Halogen, (C₁- bis C₅-)Alkyl, (C₁- bis C₅-) Alkoxy, (C₁- bis C₅-)Alkylthio, (C₁- bis C₅-) Alkylamino, (C₆- bis C₁₄-)Aryloxy, (C₆- bis C₁₄-) Aryl(C₁- bis C₅-)alkoxy, Cyano, Trifluormethyl, Carboxy, Carboxymethyl oder Carboxyethyl substituiert ist, wobei das Stickstoffatom einer Heterocycloalkyl- oder Heteroarylgruppe mit einer (C₁- bis C₅-)Alkyl-, (C₃- bis C₈-)Cycloalkyl-, (C₆- bis C₁₄-)Aryl-, (C₆- bis C₁₄-) Aryl (C₁- bis C₅-)alkyl- oder (C₁- bis C₆-)Acylgruppe substituiert sein kann, sowie die tautomeren, enantiomeren, diastereoisomeren und epimeren Formen und die pharmazeutisch verträglichen Salze, ausgenommen die Verbindung, bei der R₁ = Phenyl, R₂ = Methyl, R₃ = R₄ = H und R₅ = R₆ = Methyl sind.

2. Verbindungen mit der Formel (I) nach Anspruch 1, in welcher R₅ Wasserstoff bedeutet.

3. Verbindungen mit der Formel (I) nach Anspruch 1, in welcher R₅ und R₆ mit dem Kohlenstoffatom, an welches sie gebunden sind, einen Cyclus mit m Gliedern (m gleich 3 bis 8) bilden, der gegebenenfalls ein oder mehrere Heteroatome enthält, die aus N, O und S ausgewählt sind, und gegebenenfalls mit Amino, Hydroxy, Thio, Halogen, (C₁- bis C₅-) Alkyl, (C₁- bis C₅-) Alkoxy, (C₁- bis C₅-)Alkylthio, (C₁-bis C₅-)Alkylamino, (C₆- bis C₁₄-)Aryloxy, (C₆- bis C₁₄-) Aryl(C₁- bis C₅-)alkoxy, Cyano, Trifluormethyl, Carboxy, Carboxymethyl oder Carboxyethyl substituiert ist, oder mit dem Kohlenstoffatom einen polycyclischen C₁₀- bis C₃₀- Rest bilden, der gegebenenfalls mit Amino, Hydroxy, Thio, Halogen, (C₁- bis C₅-)Alkyl, (C₁- bis C₅-) Alkoxy, (C₁- bis C₅-)Alkylthio, (C₁- bis C₅-)Alkylamino, (C₆- bis C₁₄-)Aryloxy, (C₆- bis C₁₉-) Aryl (C₁- bis C₅-)alkoxy, Cyano, Trifluormethyl, Carboxy, Carboxymethyl oder Carboxyethyl substituiert ist.

4. Verbindungen mit der Formel (I) nach Anspruch 1, in welcher R₅ eine (C₂- bis C₂₀-)Alkylgruppe bedeutet, die mit Amino, Hydroxy, Thio, Halogen, (C₁- bis C₅-) Alkyl, (C₁- bis C₅-)Alkoxy, (C₁- bis C₅-)Alkylthio, (C₁- bis C₅-)Alkylamino, (C₆- bis C₁₄-)Aryloxy, (C₆- bis C₁₄-) Aryl(C₁- bis C₅-)alkoxy, Cyano, Trifluormethyl, Carboxy, Carboxymethyl oder Carboxyethyl substituiert ist.

5. Verbindung mit der Formel (I) nach Anspruch 1, in welcher R₅ und R₆ aus H und/oder (C₁- bis C₂₀-) Alkylgruppen ausgewählt sind, die gegebenenfalls mit Amino, Hydroxy, Thio, Halogen, (C₁- bis C₅-) Alkyl, (C₁- bis C₅-)Alkoxy, (C₁- bis C₅-)Alkylthio, (C₁- bis C₅-)Alkylamino, (C₆- bis C₁₄-)Aryloxy, (C₆- bis C₁₄-) Aryl (C₁- bis C₅-)alkoxy, Cyano, Trifluormethyl, Carboxy, Carboxymethyl oder Carboxyethyl substituiert sind.

6. Verbindung mit der Formel (I) nach einem der Ansprüche 1 bis 5, in welcher R₁ und R₂ eine Methylgruppe und R₃ und R₄ ein Wasserstoffatom bedeuten.

7. Verbindung mit der Formel (I) nach einem der Ansprüche 1 bis 6, in welcher R₁ und R₂ eine Methylgruppe und R₃ und R₄ ein Wasserstoffatom bedeuten, R₅ ein Wasserstoffatom und R₆ eine Methylgruppe bedeutet, sowie die tautomeren, enantiomeren, diastereoisomeren und epimeren Formen und die pharmazeutisch verträglichen Salze.

8. Verfahren zur Herstellung einer Verbindung nach Anspruch 1, das die Umsetzung einer Verbindung mit der allgemeinen Formel (II) 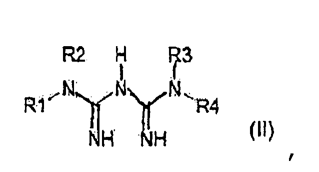 in welcher R₁, R₂, R₃ und R₄ wie zuvor definiert sind, mit einer Verbindung mit der allgemeinen Formel (III), (IV) oder (V) 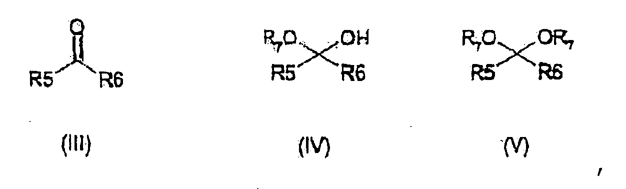 in welchen R₅ und R₆ wie zuvor definiert sind und R₇ eine Methyl- oder Ethylgruppe bedeutet, in einem polaren Lösungsmittel in Gegenwart einer organischen oder anorganischen Säure umfasst.

9. Pharmazeutische Zusammensetzung, die als Wirkstoff eine Verbindung nach einem der Ansprüche 1 bis 7 umfasst.

10. Verwendung einer Verbindung nach einem der Ansprüche 1 bis 7 zur Herstellung eines Arzneimittels, das zur Behandlung von Krankheiten bestimmt ist, die mit dem Insulinresistenzsyndrom einhergehen.

11. Verwendung einer Verbindung nach einem der Ansprüche 1 bis 7 zur Herstellung eines Arzneimittels, das zur Behandlung des Diabetes vorgesehen ist.

12. Verwendung einer Verbindung nach einem der Ansprüche 1 bis 7 zur Herstellung eines Arzneimittels, das zur Behandlung von Krankheiten vorgesehen ist, die auf die Bildung von AGEs zurückzuführen sind.

13. Verwendung einer Verbindung nach einem der Ansprüche 1 bis 7 zur Herstellung eines Arzneimittels, das zur Behandlung von Krankheiten vorgesehen ist, die aus Störungen der Nierenfunktion, Ateriosklerose, Angiopathie, Alzheimer'sche Krankheit, neurodegenerativen Erkrankungen und Senilität ausgewählt sind.
